(19) Europäisches Patentamt
European Patent Office
Office européen des brevets

(11) Numéro de publication: **0 527 768 B1**

# (12) FASCICULE DE BREVET EUROPEEN

(45) Date de publication du fascicule du brevet: **06.09.95**

(51) Int. Cl.$^{6}$: **A61K 39/40**

(21) Numéro de dépôt: **91907576.2**

(22) Date de dépôt: **03.04.91**

(86) Numéro de dépôt internationale :
**PCT/FR91/00266**

(87) Numéro de publication internationale :
**WO 91/15239 (17.10.91 91/24)**

## (54) ANTICORPS PURIFIES SPECIFIOUEMENT DIRIGES CONTRE LES LIPOPOLYSACCHARIDES (LPS) DES BACTERIES GRAM NEGATIF.

(30) Priorité: **03.04.90 FR 9004227**

(43) Date de publication de la demande:
**24.02.93 Bulletin 93/08**

(45) Mention de la délivrance du brevet:
**06.09.95 Bulletin 95/36**

(84) Etats contractants désignés:
**AT BE CH DE DK FR GB IT LI LU NL SE**

(56) Documents cités:
**EP-A- 0 271 379**

**DIAGN. MICROBIOL. INFECT. DIS., vol. 6, 1987; M.A. VANESIAN et al., pp. 11-25&NUM;**

**JOURNAL OF IMMUNOLOGICAL METHODS, vol. 129, 1990; J.W. TYLER et al., pp. 221-226&NUM;**

(73) Titulaire: **FONDATION NATIONALE DE TRANSFUSION SANGUINE**
**6, rue Alexandre Cabanel**
**F-75739 Paris Cédex 15 (FR)**

(72) Inventeur: **BASUYAUX, Bertrand**
**73, rue de Wattignies**
**F-75012 Paris (FR)**
Inventeur: **LAROZE, Alain**
**4 allée des Lacs**
**F-34170 Castelnau-le-Lez (FR)**
Inventeur: **TRINCOT, Anne**
**22, rue du Poteau**
**F-75018 Paris (FR)**

(74) Mandataire: **Warcoin, Jacques**
**Cabinet Régimbeau,**
**26, avenue Kléber**
**F-75116 Paris (FR)**

EP 0 527 768 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention se rapporte à des anticorps purifiés spécifiquement dirigés contre les lipopolysaccharides (LPS) des bactéries gram-négatif.

La surface de la membrane externe des bactéries gram négatif est essentiellement constituée de lipopolysaccharides ou LPS. La libération de ces LPS dans l'organisme humain, accrue par l'utilisation d'antibiotiques, induisant la libération d'endotoxines à partir de bactéries tuées, provoque des chocs graves voire mortels (80 000 morts aux Etats-Unis chaque année). En effet, parmi les actions biologiques des LPS on peut citer :

- une toxicité qui peut être mortelle,
- une modification de la régulation thermique,
- des troubles circulatoires etc.
- des modifications leucocytaires.

Peu de traitements existent à l'heure actuelle pour éviter ces chocs endotoxiniques, ce qui incite de nombreuses équipes à s'intéresser à toute substance pouvant jouer un rôle antagoniste à l'action de ces LPS.

La structure des LPS de toutes les bactéries gram négatif peut être schématisée ainsi. Elles présentent toutes une structure constituée d'une partie lipidique, le lipide A, responsable en grande partie des effets toxiques des LPS. Ce lipide A est rattaché à un noyau (core) par un trisaccharide le 2-céto-3-desoxyoctonate ou KDO. Sur le noyau, dont la structure polysaccharide est commune à tous les LPS, sont liées des chaînes latérales ou séquences polyosidiques qui conservent à chaque souche sa spécificité antigénique.

Des souches mutantes, dites rugueuses, ne synthétisent pas ces chaînes latérales. Leurs LPS possèdent des propriétés antigéniques dues uniquement au lipide A et au noyau.

Puisque la plupart des effets toxiques de l'endotoxine sont à associer à la partie lipidique A du LPS, il a été envisagé qu'une immunisation avec des mutants rugueux des bactéries gram négatif permettrait d'obtenir des anticorps susceptibles d'éviter les chocs endotoxiniques d'une grande variété d'organismes à gram négatif.

Les anticorps dirigés contre les LPS de ces souches rugueuses seraient dirigés contre la plupart des LPS bactériens.

Vanesian et al (Diagn. Microbiol. Infect. Dis. Vol. 6, 1987, 11-25) ont préparé des standards de dosage, composés essentiellement d'IgM anti J5 OR5 95, à partir de plasmas de donneurs sélectionnés. EP 271 379 décrit l'obtention d'Ig G monoclonales murines.

E. Ziegler, aux U.S.A., a montré, dès 1985, que l'injection de plasma de sujets vaccinés par le LPS de la souche J5 de E. coli (structure commune à toutes les bactéries gram négatif, responsable du choc septique mais sans danger pour les sujets vaccinés) entraînait une réduction significative, non pas des infections bactériennes elles-mêmes, mais des chocs septiques et de la mortalité. Toutefois, des immunoglobulines intraveineuses (obtenues par fractionnement de Cohn et traitées à la pepsine à pH 4), n'ont pas montré leur efficacité dans le traitement des chocs (le travail de Baumgartner à Lausanne : colloque à Amsterdam, 1988). En revanche, dans les études expérimentales animales, des immunoglobulines préparées à partir de plasmas sélectionnés pour leur titre en anti-LPS ont pu prévenir l'infection bactérienne chez des animaux infectés (GAFFIN et al. : Vox Sanguinis 48, 276-83, 1985).

Toutefois, pour des raisons pratiques qui sont principalement dues à des difficultés d'approvisionnement, de stockage et d'administration, l'antisérum dirigé contre E. coli J5 n'est pas approprié pour une utilisation clinique à grande échelle. En revanche, une préparation d'immunoglobulines purifiées, obtenue à partir d'un pool de plasmas de volontaires naturellement immunisés, offrirait, elle, de nombreux avantages.

Tout d'abord, une telle préparation pourrait être disponible sous une forme lyophilisée, appropriée à une longue conservation. Enfin, l'administration de cette préparation par voie intraveineuse permettrait l'injection de quantité importante en produit actif avec une perte limitée dans l'organisme.

Le but de la présente invention est précisément de répondre à cet objectif. Il vise à élaborer des préparations riches en immunoglobulines spécifiques pour des utilisations thérapeutiques et utiles tout particulièrement pour prévenir le choc septique en clinique humaine.

La technique utilisée selon l'invention pour préparer ces anticorps anti-LPS, sous une forme purifiée, consiste en une chromatographie d'immunoaffinité et met en oeuvre une immobilisation d'antigènes LPS sur un support.

Des techniques de couplage d'antigènes LPS sur des supports ont certes déjà été décrites. Toutefois aucune d'entre elles n'est réellement satisfaisante.

Il a ainsi déjà été réalisé le couplage des antigènes LPS par l'intermédiaire de leurs fonctions hydroxyles sur des fonctions époxy du support à pH très basique et donc dans des conditions dénaturantes

pour les LPS, ce qui rend la capacité de tels supports très faible voire inexploitable. (R.B. FICK et al. J. of Immunol. Method. 38 : 103-116 (1980); J. FOX et al. Infection and Immunity 20, 3 867-868 (1978)).

De même, le couplage des antigènes LPS a été effectué par les fonctions amines du ligand sur un support active comportant des fonctions cyanates et/ou imidocarbonates (gel sépharose activé au bromure de cyanogène). (E. RODAHL et al., Acta. pat. microbiol. immunol. scand. sect C, 92 : 247-254 (1984)). Or, les LPS contenant très peu de fonctions amines disponibles, se couplent difficilement à ce type de gel.

L'originalité de la technique utilisée selon la présente invention et décrite plus en détail ci-après permet, elle, d'assurer de manière efficace le couplage des antigènes LPS au support.

C'est pourquoi la présente invention concerne des anticorps purifiés spécifiquement dirigés contre les lipopolysaccharides (LPS) des bactéries gram négatif, caractérisés en ce qu'ils sont purifiés à partir d'un milieu les contenant par une étape de chromatographie d'immunoaffinité utilisant des antigènes LPS insolubilisés, support d'immunoaffinité comportant essentiellement la fraction lipide A et le noyau des LPS communs à de nombreux lipopolysaccharides de bactéries gram négatif, et en ce que après séparation des anticorps par immunoaffinié on réalise un continuel enrichissement de ces anticorps pour obtenir une fraction riche en IgM.

Il peut s'agir d'anticorps purifiés spécifiquement dirigés contre les lipopolysaccharides (LPS) des bactéries gram négatif, caractérisés en ce qu'ils sont obtenus avec un facteur d'enrichissement supérieur à 500 et de préférence supérieur à 750, en réalisant une chromatographie d'immunoaffinité des anticorps anti-LPS présents dans des liquides biologiques, ladite chromatographie mettant en oeuvre la fixation desdits anticorps sur des antigènes LPS immobilisés sur un support.

Par facteur d'enrichissement, on entend désigner le rapport activité anticorps anti-LPS par mg ou g de protéines ou d'immunoglobulines de l'éluat sur activité anticorps anti-LPS par mg ou g de protéines ou d'immunoglobulines du liquide biologique d'origine.

Les milieux contenant ces anticorps sont de préférence des liquides biologiques qui peuvent être de sources variées en particulier des plasmas ou sérums humains ou encore des plasmas ou sérums d'animaux. Il peut s'agir en particulier de fractions enrichies en IgM, dans ce cas, l'étape de purification finale ne sera pas forcément nécessaire.

Selon un mode de réalisation préféré de l'invention, les liquides biologiques utilisés ont été au préalable sélectionnés par une méthode ELISA pour leur spécificité anti-LPS. Cette méthode bien connue de l'homme du métier ne sera pas explicitée ici en détail.

La chromatographie par immunoaffinité mise en oeuvre selon l'invention repose donc sur l'utilisation d'antigènes LPS insolubilisés qui retiennent les immunoglobulines du mélange dirigées contre eux. Ces immunoglobulines sont ensuite récupérées après dissociation des complexes antigène-anticorps.

L'originalité de la technique proposée tient à la transformation des groupements hydroxyles du LPS en groupements aldéhydes très réactifs qui permettent un couplage soit directement sur une résine portant des fonctions hydrazines ou amines (aminohexyl sépharose®, hydrazide d'acide adipique sépharose®, lysine sépharose®, gélatine sépharose®) soit sur une protéine, par exemple l'albumine d'origine humaine ou animale, qui sera elle-même couplée sur une résine activée, du sépharose® activé au bromure de cyanogène par exemple.

Cette méthode a ainsi l'avantage de laisser accessible le lipide A des LPS, c'est-à-dire la partie antigénique commune à tous les LPS.

Les immunoglobulines présentes dans le liquide biologique à purifier sont donc retenues par ces antigènes LPS. Les anticorps fixés aux antigènes sont ensuite lavés de façon à éliminer la plupart des molécules ne reconnaissant pas l'antigène puis récupérés par dissociation des complexes antigène-anticorps.

Cette étape de dissociation, l'élution, est une étape importante de l'immunopurification. L'élution est le plus souvent réalisée à l'aide d'un agent chimique, qui modifie les propriétés physico-chimiques de la solution, permettant ainsi la dissociation des complexes antigènes-anticorps, sans pour autant altérer les propriétés biologiques des antigènes et des anticorps spécifiques.

L'élution à pH acide est la plus utilisée. Le pH doit être suffisamment acide pour garantir un bon rendement d'élution mais pas trop acide pour ne pas être préjudiciable aux anticorps. Il sera de préférence de l'ordre de 2,5. On peut ainsi utiliser des tampons glycine-HCl et acétate de sodium.

De préférence, l'antigène LPS immobilisé sur le support provient d'un mutant d'une bactérie gram négatif, dépourvu de chaînes latérales polyosidiques et constitué uniquement de lipide A et d'un noyau commun a de nombreux lipopolysaccharides de bactéries gram négatif. Ce mutant possède ainsi des propriétés antigéniques communes à de nombreux LPS.

Il peut ainsi s'agir du mutant J5 d'E. coli O 111 ou du mutant Re de Salmonella minnesota 595.

Selon un mode privilégié de l'invention, on utilise le mutant J5 dont la structure est la suivante :

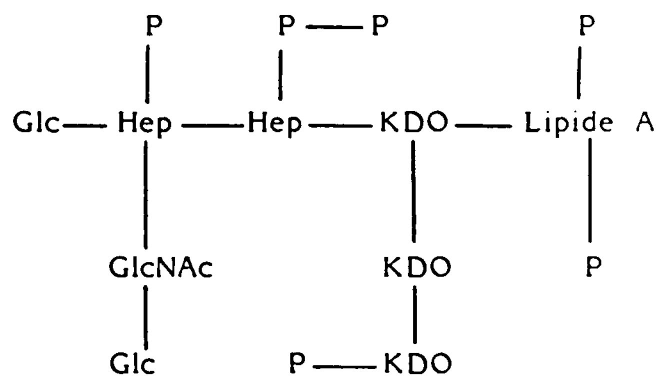

Ce mutant J5 d'E.Coli ne présente pas de chaîne O-spécifique et, son nucleus est d'autre part simplifié. Il est de ce fait un déterminant antigénique commun à de nombreux LPS naturels et reconnaît les anticorps dirigés contre le lipide A et le noyau.

A la fin de la chromatographie par immunoaffinité, l'ensemble de l'activité anti-LPS se trouve alors concentré dans l'éluat : en une seule étape il est ainsi possible de purifier les anticorps d'un facteur d'environ 500 à 1 000 fois.

La présente invention concerne plus particulièrement les compositions à base d'IgM, notamment les IgM anti-LPS de bactéries gram négatif dirigés contre le fragment lipide A - noyau des LPS communs à de nombreux LPS de bactéries. En effet, les exemples suivants démontrent que l'activité thérapeutique est essentiellement portée par les IgM.

Compte tenu de leur pureté protéique, les anticorps anti-LPS, objets de la présente invention peuvent être administrés à l'animal et/ou à l'homme, seuls ou en combinaison avec un autre composé dans une composition pharmaceutique. Ils sont tout particulièrement utiles dans la prévention ou le traitement des chocs endotoxiniques.

La présente invention se rapporte donc également aux compositions pharmaceutiques contenant au moins à titre de principe actif, des anticorps anti-LPS selon l'invention.

Les exemples et figures donnés ci-après, à titre non limitatif, permettront de mettre en évidence d'autres caractéristiques et avantages de la présente invention.

La figure 1 décrit l'activité anti-LPS d'un concentré d'anticorps anti-LPS purifiés, objet de la présente invention.

Exemple 1 : Immunopurification d'anticorps anti-LPS

1) Matériel et méthodes

1.1. Préparation du support

La réactivité chimique des LPS a été rendue possible par clivage oxydatif au periodate de sodium des alphadiols portés par le KDO (le trisaccharide 2-ceto-3-désoxyoctonate, reliant le nucléus et le lipide A) et les oses du nucléus (heptose et glucose), avec formation de fonctions aldéhydes (réaction de Malprade). Ces fonctions aldéhydes peuvent se condenser sur des fonctions amines ou hydrazines (réaction de Schiff) provenant soit d'un support chromatographique (par exemple hydrazide Sépharose®), soit d'une protéine (albumine sérique bovine). La fonction imine formée peut être ensuite stabilisée par réduction au borohydrure de sodium ou au cyanoborohydrure de sodium.

**a)** Activation des LPS E.Coli J5

Dans un tube Falcon 50 ml (Becton Dickinson) en polypropylène, sont introduits successivement 35 mg de LPS E. Coli J5 (List Biological Laboratories) en solution dans de l'eau pour préparations injectables (soit environ 8 micromoles) et 0,35 ml d'une solution de 2,15 g de $NaIO_4$ dans 100 ml d'eau PPI (soit 35 micromoles). Le tube est ensuite agité 24 heures, dans l'obscurité et à température ambiante.

**b)** Couplage des LPS sur gel d'Hydrazide d'Acide Adipique-Sépharose®

25 ml de gel sont lavés sur verre fritté avec dans un premier temps, 1 litre d'eau PPI, puis 500 ml de tampon acétate 100 mM pH5. Dans un flacon de 250 ml en verre, on introduit le gel lavé et essoré, les 11 ml de la solution de LPS activés et environ 80 ml de tampon acétate. Le tube est agité 24 heures, à température ambiante dans l'obscurité.

Le gel est ensuite essoré et lavé sur verre fritté successivement avec 200 ml de tampon acétate et 200 ml d'une solution de NaCl 2M puis équilibré en tampon phosphate 100 mM pH 7,5.

1.2. Essai d'immunopurification

**a)** Chromatographie

L'essai a été réalisé sur une colonne IBF 25 contenant 30 ml de gel susmentionné. Outre la colonne, le système chromatographique se compose de :

. une pompe péristaltique (Gilson, type minipuls 2),
. un détecteur UV, DO : 280 nm (Pharmacia, S.P.M. UV-1), équipé d'une cellule HR-10,
. un enregistreur (Pharmacia, REC-482).

Avant l'injection de la source d'anticorps dans la colonne, un essai à blanc est effectué :

. lavage du gel avec 150 ml de tampon phosphate 0,1 M pH 7,5,
. lavage du gel avec 150 ml de tampon glycine-HCl 0,1 M pH 2,5,
. rééquilibrage du gel avec 150 ml de tampon phosphate 0,1 M pH 7,5.

La source à purifier est du plasma humain (600 ml) sélectionné par méthode ELISA pour sa spécificité anti-LPS. Ce plasma est dilué au demi dans du tampon phosphate 0,1 M pH 7,5 (soit 1 200 ml total).

Les 1 200 ml de plasma dilué (25 $g/l^{-1}$ de protéines, titre en ELISA au 1/53$^{e}$) ont été injectés dans la colonne à un débit de 60 ml par heure, assurant un temps de séjour de 30 minutes. Après lavage avec du tampon phosphate 0,1 M pH 7,5, les anticorps spécifiques sont élués par du tampon glycine-HCl pH 2,5. L'éluat est neutralisé extemporanément avec 5 ml de tampon phosphate 0,5 M pH 7,5.

**b)** Suivi analytique

- Dosages des classes d'immunoglobulines G, M et A dans l'éluat.

Ils sont effectués par néphelémétrie.

On utilise un analyseur immunochimique (Immunochemistry Analyser ICS-II Beckman). Cet appareil mesure l'intensité de la lumière déviée par des particules en suspension dans une cellule de détection. Ces particules proviennent de la réaction d'immunoprécipitation entre l'antigène que l'on veut doser, et l'anticorps. Selon le calibrateur et l'anticorps utilisés, on peut doser les classes d'immunoglobulines humaines, G, A, ou M, l'albumine ou la transferrine humaines. La précision est de l'ordre de 5 % au centre de la gamme, de 10 % aux faibles concentrations.

- Dosage de la concentration protéique de l'éluat.

La concentration a été évaluée à l'aide du dosage par BCA (acide bicinchoninique) (Kit PIERCE).

Selon le principe de cette méthode, les protéines provoquent en milieu alcalin la réduction de l'ion cuivrique ($Cu^{2+}$) selon la réaction du Biuret. La réaction entre le sel de sodium de l'acide bicinchoninique (BCA) et l'ion cuivreux est sensible et très spécifique. Le complexe formé BCA-$Cu^{+}$ est soluble dans l'eau et montre une forte absorbance à 562 nm.

Le dosage est réalisé de la manière suivante :

. 1 part de solution à 4 % de sulfate de cuivre et 50 parts d'une solution de carbonate, bicarbonate, tartrate de sodium, de réactif BCA dans NaOH 0,2 M sont ajoutés à 0,1 ml de solution à doser ou 0,1 ml de tampon de dilution des échantillons pour le blanc + 2 ml de réactif, l'ensemble est agité, incubé 2 heures à température ambiante ou 30 minutes à 37 °C puis révélé à 562 nm contre le blanc de lecture ;
. chaque échantillon est dosé en triple ; on réalise une gamme étalon de SAB (Pierce) et d'une préparation d'immunoglobulines humaines de 0,05 à 1 mg/ml.

- Electrophorèse sur gel de polyacrylamide 4/30 (Pharmacia) en présence de SDS
- Dosage de l'activité anti-LPS dans la source, le filtrat et l'éluat

Il a été réalisé comme suit:

. adsorber pendant 16 heures à température ambiante sur les puits de plaques de microtitration en PVC (Falcon réf. 3912, Becton Dickinson), les LPS E. coli J5 (List Biological Laboratories) à raison de

0,1 ml par puits d'une solution de LPS à 10 µg/ml dans du PBS contenant 1,6% (p/v) de $MgCl_2$ et 0,01% (p/v) de merthiolate;

. après 3 lavages avec une solution à 0,9% (p/v) NaCl et 0,1% (v/v) Tween® 20, les puits sont saturés pendant 1 heure à température ambiante avec 150 µl d'une solution d'albumine sérique bovine à 5% (p/v) dans du PBS.

. après 3 lavages avec une solution à 0,9% (p/v) NaCl et 0,1% (v/v) Tween® 20, incuber 3 heures à 37°C les échantillons à doser à différentes dilutions dans du PBS à 5% (p/v) d'albumine sérique bovine.

. après 5 lavages avec une solution à 0,9% (p/v) NaCl et 0,1% (v/v) Tween® 20, incuber 90 minutes à 37°C avec 100 µl d'une solution d'immunoglobulines de chèvre anti-immunoglobulines G + M humaines couplées à la peroxydase, diluées au 1/400 000 dans du tampon phosphate 0,1 M pH 7,5 avec 0,1% (p/v) d'albumine sérique bovine.

. après 5 lavages avec une solution à 0,9% (p/v) NaCl et 0,1% (v/v) Tween® 20, l'activité enzymatique est révélée par addition d'orthophénylène diamine dans $H_2O_2$ 0,03% (Diagnostics Pasteur) pendant 20 minutes à température ambiante en l'absence de lumière (100 µl de substrat par puits); la réaction enzymatique est arrêtée par ajout de $H_2SO_4$ 4N (50 µl par puits). La densité optique de chaque puits est déterminée à 492 nm, à l'aide d'un lecteur de microplaque ELISA (Molecular Devices, Vmax).

2) **Résultats**

Le tableau I présenté ci-après et la figure 1 rendent compte des résultats obtenus.

TABLEAU 1

| CONCENTRATION (g/l) | |
|---|---|
| - Protéines (BCA)<br>- IgG (néphélémétrie)<br>- IgM (néphélémétrie)<br>- IgA (néphélémétrie)<br>- Ig totales (IgG + IgM + IgA) | 0,61<br>0,26<br>0,33<br>0,04<br>0,63 |
| TITRE | 1/1050$^{e}$ |
| PURETE ELECTROPHORETIQUE des Ig totales | 90% |

Les concentrations protéiques, correspondant au titre (dilution de l'échantillon qui donne un signal égal à la moitié de la densité optique maximale, soit 1,3/2 = 0,65) du plasma de départ et de l'éluat. sont respectivement de :

$$\frac{0{,}1\,ml \times 25\,mg/ml}{53} = 0{,}0472\,mg = 47{,}2\,\mu g$$

$$\frac{0{,}1\,ml \times 0{,}63\,mg/ml}{1\,050} = 0{,}00006\,mg = 60\,ng$$

soit une "différence" de

$$\frac{47\ 200\ mg}{60\ mg} = 787;$$

cette valeur peut être assimilée à un coefficient d'enrichissement.

On peut évaluer le rendement de la purification par le biais du rapport :

$$\frac{(\text{titre x volume})\ \text{éluat}}{(\text{titre x volume})\ \text{plasma de départ}} \times 100$$

$$\text{soit}\ \frac{1\ 050 \times 30}{53 \times 1\ 200} \times 100 \ \# \ 50\%$$

D'une part cette valeur est toute relative, d'autre part la perte en anticorps n'est pas dramatique, dans la mesure où elle est dûe principalement aux anticorps de faible affinité, peu intéressants pour les applications envisagées.

**Exemple 2 : Préparation et caractérisation d'un mélange concentré d'IgG et d'IgM plasmatiques humains dirigés contre les LPS**

**1) Matériel et méthodes**

1.1. Préparation d'un mélange IgG/IgM anti-LPS

Vingt quatre éluats d'immunopurification sont décongelés à température ambiante. Le mélange est ensuite soumis à une diafiltration sur membrane YM 100 (76 mm de diamètre, seuil de coupure 100 kD), (AMICON) avec du tampon phosphate 20 mM pH7 contenant 0,9 % (p/v) de NaCl. Lorsque le volume total du mélange se trouve abaissé à une valeur d'environ 40 ml, celui-ci est prélevé et filtré successivement sur filtre Minisart 5µm puis 0,22 µm (SARTORIUS).

Comme décrit précédemment, les classes d'immunoglobulines G, M et A sont dosées par néphélémétrie à l'aide d'un analyseur immunochimique Beckman ICS-11.

1.2. Caractérisation en ELISA du mélange IgG/IgM anti LPS

La spécificité du mélange immunopurifié et concentré d'immunoglobulines polyclonales humaines anti LPS a été évaluée en ELISA, en comparaison avec celle des immunoglobulines polyvalentes intraveineuses obtenues par fractionnement de Kistler-Nitschmann modifié, de pools plasmatiques d'au moins 1000 donneurs de sang (Polygamma de Bio Transfusion, France), vis-à-vis du LPS de E. coli J5 (Eurobio) et d'autres bactéries gram négatif : E. coli O111 : B 4, Salmonella minnesota R5, Klebsiella pneumoniae, Pseudomonas aeruginosa (Sigma).

Le protocole utilisé pour ces évaluations est identique à celui décrit précédemment (exemple 1).

**2) Résultats**

La concentration des classes IgG, IgM et IgA dans le mélange anti-LPS est de 6,1, 3,1, et 0,9 g/l respectivement.

Le mélange anti-LPS reconnait les LPS des 4 souches sauvages testées (Tableau 2). Les titres mesures pour les LPS des souches sauvages sont un peu plus faibles que celui obtenu vis-à-vis de la souche mutante E Coli J5. Ceci s'explique, d'une part par le mode de préparation de ces anticorps, immunopurifiés sur E Coli J5 immobilisés, d'autre part par le fait que ces anticorps ne peuvent se fixer que sur le noyau des LPS, lequel se trouve plus ou moins accessible en fonction de l'importance des chaînes latérales polyosidiques sur les LPS des souches sauvages.

## TABLEAU 2

| LPS | Concentrations protéiques $nC_{50}$ correspondant au titre, donnant un signal égal à la moitié de la densité optique maximale (n = nombre de sites de liaison avec l'antigène). | | Enrichissement = |
|---|---|---|---|
| | $nC_{50}$ de la Polygamma (mgl-1) | $nC_{50}$ du mélange (mgl-1) | Rapport $\frac{nC_{50}\ \text{Polygamma}}{nC_{50}\ \text{mélange}}$ |
| E. coli J5 | 333 | 3 | 111,0 |
| E. coli 0111 : B4 | 125 | 17 | 7,4 |
| S. minnesota R5 | 333 | 10 | 33,3 |
| K. pneumoniae | 250 | 10 | 25,0 |
| P. aeruginosa | 833 | 20 | 41,7 |

Le produit Polygamma reconnaît également les LPS des souches utilisées ; mais dans le cas présent tous les anticorps sont pris en compte, à la fois ceux dirigés contre le noyau et ceux dirigés contre les chaînes latérales.

Malgré la perte supposée des anticorps dirigés contre les chaînes latérales des LPS lors de l'étape d'immunopurification sur E. coli J5 immobilisés, un enrichissement en activité anti-LPS peut être constaté dans le mélange par rapport au produit Polygamma. Cet enrichissement est bien entendu mis en évidence pour le LPS de E. coli J5 mais aussi pour ceux de Salmonella minnesota R5, Klebsiella pneumoniae et Pseudomonas aeruginosa, et dans une proportion plus modeste pour le LPS de E. coli 0111 : B4, souche pour laquelle la réponse anticorps naturelle chez l'homme semble plus significativement dirigée contre les

chaînes latérales.

**Exemple 3 : Activité pharmacologique in vitro du mélange concentré IgG/IgM anti-LPS**

**1/ Détermination de l'activité anti-LPS du mélange immunopurification IgG/IgM à l'aide d'un essai d'inhibition de multiplication de virus bactériophages**

**1.1.) Matériel et méthodes**

Le virus bactériophage Ø X 174 tient son infectivité vis-à-vis de l'hôte E. coli de la reconnaissance spécifique des lipopolysaccharides des parois de E. coli en présence d'ions Ca2+ (A.M. FIELD, E. ROWAIT et R.J.P. WILLIAMS in Biochem J. 263, 695-702 (1989). Nous avons donc mis en oeuvre cette propriété dans un essai d'inhibition de multiplication des virus Ø X 174 en ajoutant des immunoglobulines ayant un titre anti-LPS de E coli. Le titrage des phages est réalisé par dilutions décimales du stock primaire de virus dans le diluant contenant la souche sensible. Les dilutions sont mises à incuber en présence de gélose de surface liquide, puis versées sur les boîtes de Pétri contenant la gélose nutritive en profondeur.

Après incubation 18 à 24h à 37 °C, les boîtes sont lues selon le nombre de plages de lyse obtenues.

Les titres sont exprimés en unités formant plages (UFP/ml).

L'activité du mélange immunopurifié concentré IgG/IgM anti-LPS (cf exemple 2) a été évaluée sur ce modèle en comparaison avec celle des immunoglobulines polyvalentes intraveineuses (Polygamma de Bio Transfusion).

**1.2.) Résultats**

Par rapport au titre initial de virus dans le stock primaire (6 X $10^7$ UFP/ml) on constate un effet inhibiteur de multiplication de 3,14 log pour une solution à 10 mg/ml d'immunoglobulines non spécifiques.

L'effet inhibiteur s'estompe avec la dilution, puisqu'à 0,625 mg/ml on ne mesure plus qu'une différence de 1,48 Log (tableau 3).

TABLEAU 3

| Inhibition de la Multiplication de Ø X 174 en fonction de la concentration en Polygamma | | |
|---|---|---|
| Concentration en Polygamma (mg/ml) | Titre : (N) en UFP/ml | log N |
| 10 | 4,50 $10^4$ | 4,65 |
| 5 | 1,00 $10^5$ | 5,00 |
| 2,5 | 1,50 $10^5$ | 5,18 |
| 1,25 | 1,42 $10^6$ | 6,15 |
| 0,625 | 2,04 $10^6$ | 6,31 |

L'activité inhibitrice de la multiplication des phages O x 174 en fonction de la concentration du mélange de IgG/IgM anti LPS est exprimée dans le tableau 4.

TABLEAU 4

| Inhibition de la multiplication de Ø x 174 en fonction de la concentration en mélange IgG/IgM anti-LPS | | |
|---|---|---|
| Concentration en mélange IgG/IgM anti-LPS (mg/ml) | Titre (N) en UFP/ml | log N |
| 10 | 2,85 $10^3$ | 3,45 |
| 5 | 5,55 $10^3$ | 3,74 |
| 2,5 | 6,80 $10^3$ | 3,83 |
| 1,25 | 1,13 $10^4$ | 4,05 |
| 0,625 | 2,34 $10^4$ | 4,37 |

L'effet inhibiteur est très significativement plus important dans le cas du mélange immunopurifié IgG/IgM anti-LPS que dans celui du produit Polygamma, en particulier aux concentrations 1,25 mg/ml et 0,625 mg/ml où la différence d'activité est d'un ordre de 100 (2 log) ; Ainsi une activité anti LPS portée par des anticorps se trouve beaucoup plus concentrée dans le mélange immunopurifié que dans les immunoglobulines polyvalentes intraveineuses (Polygamma) ; ladite activité se traduit par une fixation des anticorps sur les LPS des parois des bactéries en culture, fixation très préjudiciable à la reconnaissance de la paroi bactérienne par le phage.

**2/ Détermination de l'activité anti-LPS du mélange immunopurifié IgG/IgM, à l'aide d'un essai d'inhibition de la secrétion de TNF & par des macrophages péritoneaux murins sensibilisés aux LPS.**

**2.1.) Matériel et méthodes**

Sous l'action des endotoxines bactériennes, les monocytes/macrophages sécrètent du TNFα et d'autres monokynes (IL 1, IL6...) ; le TNFα est donc un bon marqueur de l'action des endotoxines.

Les macrophages péritonéaux murins sont prélevés chez 3 souris BALB/C ayant reçu 7 jours auparavant 0,5 ml d'adjuvant complet de Freund par voie intrapéritonéale. Ainsi, 5 ml de milieu complet RPMI 1640 supplémenté en glutamine, antibiotiques et ultroser® HY 1 % (v/v) (IBF) sont injectés dans la cavité péritonéale de chaque souris, puis réaspirés et mélangés.

Après 3 lavages en milieu de Hanks, le culot cellulaire est remis en suspension dans le milieu complet. On obtient environ 150 millions de cellules dont 60 millions de macrophages. Une solution ajustée à $10^6$ macrophages par ml (2,5 $10^6$ cellules péritonéales/ml) dans le milieu complet est preparée, 100 µl de cette solution sont distribués dans des puits de microplaques (96 puits). Apres incubation 3 heures à 37 °C dans l'incubateur à $CO_2$, les surnageants sont éliminés : seuls les macrophages restent adsorbés sur le fond des micropuits. On ajoute alors 100 µl d'anticorps à tester (ou 100 µl de milieu complet seul) dilués dans le milieu complet puis 100 µl de LPS dilués également dans le milieu complet. Trois puits sont consacrés par dilution d'anticorps et par dilution de LPS.

Après 24 heures d'incubation, les surnageants sont prélevés : les 3 surnageants de chaque "situation" sont mélangés pour disposer ainsi d'assez de volume pour être dosés en ELISA. Les surnageants sont ensuite congelés à -40 °C et le dosage du TNF alpha est réalisé en différé. Le dosage du TNF alpha murin est réalisé à l'aide du kit ELISA produit par les Laboratoires GENZYME et commercialisé par la société TEBU. Il s'agit d'un dosage de type sandwich avec un anticorps monoclonal de hamster anti-TNF alpha murin adsorbé sur les microplaques. Après incubation avec le TNF alpha, on incube avec des anticorps polyclonaux de chèvre anti-TNF alpha murin. Enfin, après incubation avec des anticorps polyclonaux d'âne anti-immunoglobulines de chèvre marqués à la peroxydase, les immuncomplexes sont révélés par l'action de la peroxydase sur de l'orthophénylène diamine et lecture de la densité optique à 492 nm.

Deux souches de LPS ont été utilisées : le LPS de E. coli J5 et le LPS de E. coli 055:B5 (souche sauvage).

La préparation d'anticorps IgG/IgM immunopurifiés anti-LPS a été évaluée quant à son pouvoir d'inhiber la secrétion de TNFα par les macrophages sensibilisés aux LPS susmentionnés, en comparaison avec le produit Polygamma (Bio Transfusion).

**2.2.) Résultats**

Les résultats obtenus sont résumés figure 2 pour le mélange anti-LPS et figure 3 pour le Polygamma (symbolisée par GVP pour gamma polyvalentes intraveineuses).

Le mélange anti-LPS inhibe très significativement et de manière dose dépendante l'action du LPS de E. coli J5 comme celle du E. coli 055:B5.

Ainsi ces anticorps immunopurifiés sur E. coli J5 immobilisés (souche mutante dépourvue de chaînes latérales) sont capables de neutraliser très efficacement dans un système d'évaluation biologique in vitro l'action de LPS de souches sauvages. Ces anticorps montrent ainsi clairement leur intérêt potentiel pour la prévention et/ou le traitement du choc endotoxinique.

En revanche, le produit Polygamma n'inhibe que partiellement l'action des LPS, et ce à des doses au moins 100 fois supérieures à celles du mélange immunopurifié anti-LPS.

**Exemple 4 : Préparation et caractérisation d'un concentré d'IgM plasmatiques humaines et d'un concentré d'IgG plasmatiques humaines, dirigées contre les LPS**

**1) Matériel et méthodes**

1.1. Préparation de chacun des 2 concentrés

Le mélange concentré d'IgM/IgG obtenu et décrit précédemment (exemple 2) est reconcentré dans un volume de 4 ml avant d'être chromatographié par tamisage moléculaire sur gel ACA 34 (IBF). Les classes d'immunoglobulines (G, M, A) sont dosées en néphélémétrie.

1.2. Caractérisation en ELISA de chacune des préparations IgM et IgG anti-LPS

La spécificité des deux fractions a été évaluée en ELISA, en comparaison avec celle du mélange initial immunopurifié IgG/IgM anti-LPS et avec celle des immunoglobulines polyvalentes intraveineuses (Polygamma de Bio Transfusion), vis-à-vis du LPS de E. Coli J5 (Eurobio) et d'autres bactéries gram négatif : E. coli 0111 : B4, Salmonella minnesota R5, Klebsiella pneumoniae, Pseudomonas aeruginosa (Sigma). Le protocole utilisé pour ces évaluations est identique à celui décrit précédemment (exemple 1).

**2) Résultats**

Les concentrations des concentrés IgM et IgG anti-LPS en IgM, IgG et IgA sont de 9, 0,7 et 1 g/l pour le concentré en IgM, et 0,8, 12,5 et 0,4 g/l pour le concentre en IgG.

Les IgM et les IgG anti-LPS préparées reconnaissent en ELISA les LPS des 4 souches sauvages testées. En apparence, les IgG semblent montrer un titre un peu plus important, mais compte tenu du fait que les IgM ne peuvent pas se lier à l'antigène adsorbé sur phase solide par tous ses sites de liaison, il est difficile de tirer une conclusion plus précise.

**Exemple 5 : Détermination de l'activité anti-LPS de préparations d'IgM plasmatiques humaines et d'IgG plasmatiques humaines dirigés contre les LPS**

Le protocole utilisé est identique à celui exposé dans l'exemple 3 (2.1).

Deux souches de LPS ont été utilisées dans ce modèle : le LPS de E. coli J5 et le LPS de E. coli 055 : B5.

Les résultats obtenus sont résumés figure 4 (IgM) et figure 5 (IgG).

La préparation d'IgM anti-LPS inhibe très significativement et de manière dose dépendante l'action des LPS E. coli J5 et E. coli 055 : B5. Les IgM anti-LPS sont ainsi capables de neutraliser très efficacement dans un système d'évaluation biologique in vitro l'action de LPS de souches sauvages. Les IgM montrent ainsi clairement leur intérêt potentiel pour la prévention et/ou le traitement du choc endotoxinique.

En revanche, la préparation d'IgG anti-LPS n'inhibe que partiellement l'action des LPS. Peut-être même les IgM résiduelles pourraient expliquer partiellement, voire totalement l'effet de la préparation d'IgG.

Ainsi l'activité neutralisante des IgM polyclonales anti-LPS est très supérieure à celle des IgG polyclonales, peut-être en raison du poids moléculaire important des IgM et des nombreux sites de fixation de l'antigène présents sur l'IgM, permettant ainsi à l'IgM de piéger efficacement les LPS qui sont des structures assez volumineuses.

**Revendications**

1. Composition pharmaceutique caractérisée en ce qu'elle contient un mélange d'anticorps purifiés spécifiquement dirigés contre les lipopolysaccharides (LPS) des bactéries gram négatif, et en ce que lesdits anticorps sont constitués d'un mélange d'IgM, IgG et/ou IgA, majoritairement anti-lipide A, susceptibles d'être purifiés à partir de plasma ou sérums humains, par une étape de chromatographie d'immunoaffinité utilisant des antigènes LPS insolubilisés, le support d'immunoaffinité comportant essentiellement le noyau des LPS communs à de nombreux lipopolysaccharides de bactéries gram négatif, lesdits antigènes LPS étant immobilisés sur le support de manière à laisser accessible leur lipide A auxdits anticorps, par couplage de leurs fonctions aldéhydes, issues de la transformation de leurs fonctions hydroxyles, avec des fonctions hydrazines ou amines,
et en ce qu'après séparation des anticorps par immunoaffinité on réalise un enrichissement de ces

anticorps pour obtenir une fraction riche en IgM.

2. Composition pharmaceutique selon la revendication 1, caractérisée en ce que l'antigène LPS utilisé provient d'un mutant d'une bactérie gram négatif dépourvu de chaînes latérales polyosidiques et constitué d'un noyau commun à de nombreux lipopolysaccharides et en particulier de lipide A.

3. Composition pharmaceutique selon l'une des revendications 1 ou 2, caractérisée en ce que l'antigène LPS provient du mutant J5 d'E. coli 0111.

4. Composition pharmaceutique selon l'une des revendications 1 à 3, caractérisé en ce que les antigènes sont couplés soit directement sur une résine portant des fonctions hydrazines ou amines, soit sur les fonctions amines d'une protéine, elle-même liée à un support.

5. Composition pharmaceutique selon la revendication 4, caractérisée en ce que l'antigène LPS est couplé à du gel d'agarose sur lequel sont fixées des fonctions hydrazines ou amines.

6. Composition pharmaceutique selon l'une des revendications 1 à 5, caractérisée en ce que les anticorps présentent une pureté protéique supérieure ou égale à 50%.

7. Composition pharmaceutique selon l'une des revendications 1 à 6, caractérisée en ce qu'elle est utile pour la prévention ou le traitement des chocs endotoxiniques.

**Claims**

1. Pharmaceutical composition characterized in that it contains a mixture of purified antibodies specifically directed against the lipopolysaccharides (LPS) of Gram-negative bacteria, and in that the said antibodies consist of a mixture of IgM, IgG and/or IgA, predominantly against lipid A, capable of being purified from human plasma or sera by an immunoaffinity chromatography step using insolubilized LPS antigens, the immunoaffinity support essentially containing the core of LPSs which are common to numerous lipopolysaccharides of Gram-negative bacteria, the said LPS antigens being immobilized on the support so as to leave their lipid A accessible to the said antibodies by coupling of their aldehyde functional groups, derived from the conversion of their hydroxyl functional groups with hydrazine or amine functional groups, and in that after separation of the antibodies by immunoaffinity, enrichment of these antibodies is carried out in order to obtain an IgM-rich fraction.

2. Pharmaceutical composition according to Claim 1, characterized in that the LPS antigen used is obtained from a mutant of a Gram-negative bacterium lacking polyglycosidic side chains and consisting of a core common to numerous lipopolysaccharides and in particular of lipid A.

3. Pharmaceutical composition according to either of Claims 1 and 2, characterized in that the LPS antigen is obtained from the J5 mutant of E. Coli 0111.

4. Pharmaceutical composition according to one of Claims 1 to 3, characterized in that the antigens are coupled either directly to a resin carrying hydrazine or amine functional groups or to the amine functional groups of a protein which is itself bound to a support.

5. Pharmaceutical composition according to Claim 4, characterized in that the LPS antigen is coupled to agarose gel on which hydrazine or amine functional groups are fixed.

6. Pharmaceutical composition according to one of Claims 1 to 5, characterized in that the antibodies exhibit a protein purity greater than or equal to 50%.

7. Pharmaceutical composition according to one of Claims 1 to 6, characterized in that it is useful for the prevention or treatment of endotoxin shocks.

**Patentansprüche**

1. Pharmazeutische Zusammensetzung, dadurch gekennzeichnet, daß sie ein Gemisch von gereinigten Antikörpern enthält, die spezifisch gerichtet sind gegen die Lipopolysaccharide (LPS) von Gram-negativen Bakterien und daß die genannten Antikörper bestehen aus einem Gemisch von IgM, IgG und/oder IgA, das überwiegend ein Anti-Lipid A ist, die gereinigt werden können, ausgehend von Humanplasma oder Humanserum, durch eine Immunoaffinitäts-Chromatographie-Stufe unter Verwendung der insolubilisierten Antigene LPS, wobei der Immunoaffinitäts-Träger im wesentlichen den Kern der LPS enthält, die zahlreichen Lipopolysacchariden von Gram-negativen Bakterien gemeinsam sind, wobei die genannten Antigene LPS in der Weise auf dem Träger immobilisiert sind, daß ihr Lipid A für die genannten Antikörper zugänglich bleibt, durch Kuppeln ihrer Aldehyd-Funktionen, die aus der Transformation ihrer Hydroxyl-Funktionen stammen, mit Hydrazin- oder Amin-Funktionen, und daß nach der Abtrennung der Antikörper durch Immunoaffinitäts-Chromatographie eine Anreicherung dieser Antikörper erzielt wird unter Bildung einer an IgM reichen Fraktion.

2. Pharmazeutische Zusammensetzung nach Anspruch 1, dadurch gekennzeichnet, daß das verwendete Antigen LPS aus einer Mutante eines Gram-negativen Bakteriums stammt, die frei von polyosidischen Seitenketten ist und aus einem Kern besteht, der zahlreichen Lipopolysacchariden gemeinsam ist, und insbesondere dem Lipid A.

3. Pharmazeutische Zusammensetzung nach einem der Ansprüche 1 oder 2, dadurch gekennzeichnet, daß das Antigen LPS aus der Mutante J5 von E. coli 0111 stammt.

4. Pharmazeutische Zusammensetzung nach einem der Ansprüche 1 bis 3, dadurch gekennzeichnet, daß die Antigene entweder direkt an ein Harz gekuppelt sind, das Hydrazin- oder Aminfunktionen trägt, oder an die Aminfunktionen eines Proteins, das selbst an einen Träger gebunden ist.

5. Pharmazeutische Zusammensetzung nach Anspruch 4, dadurch gekennzeichnet, daß das Antigen LPS an Agarosegel gekuppelt ist, an dem Hydrazin- oder Aminfunktionen fixiert sind.

6. Pharmazeutische Zusammensetzung nach einem der Ansprüche 1 bis 5, dadurch gekennzeichnet, daß die Antikörper eine Protein-Reinheit von $\geqq$ 50 % aufweisen.

7. Pharmazeutische Zusammensetzung nach einem der Ansprüche 1 bis 6, dadurch gekennzeichnet, daß sie verwendbar ist für die Prävention oder Behandlung von Endotoxin-Schocks.

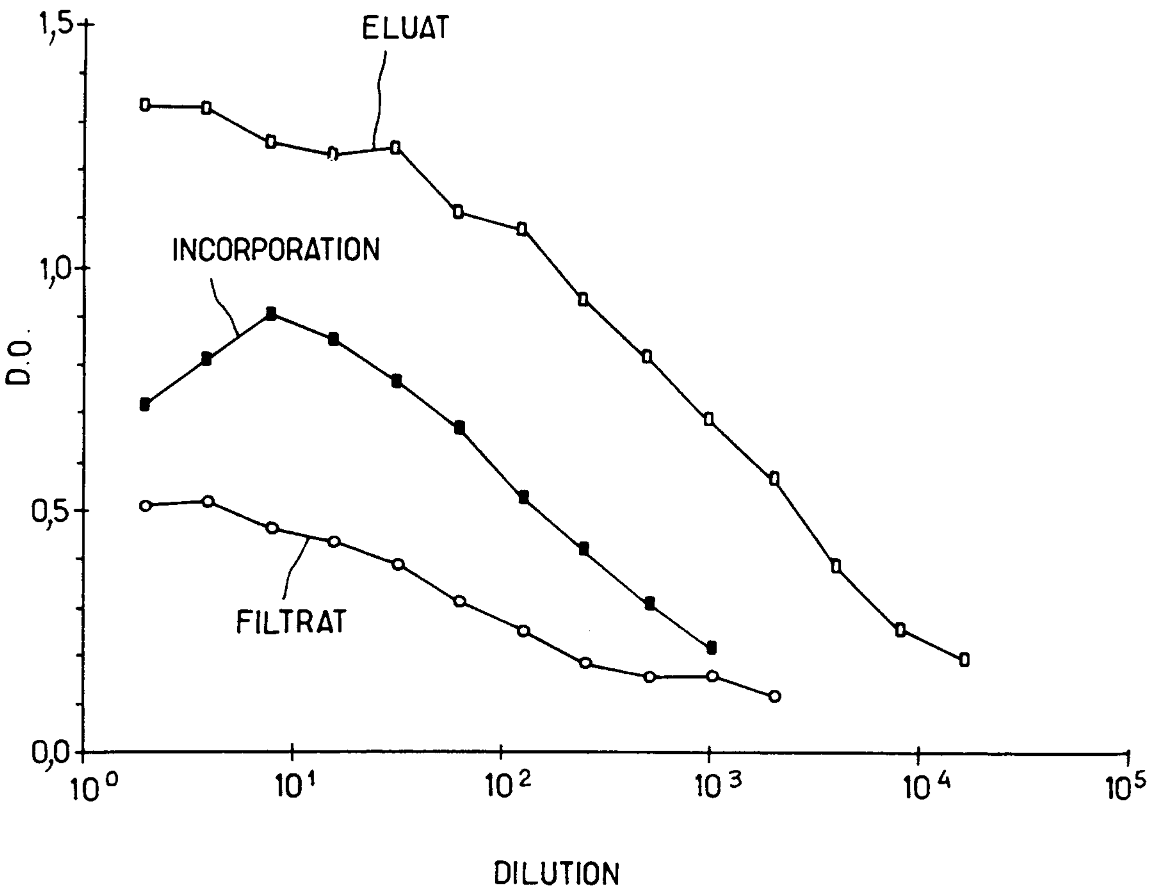
1,5
1,0
0,5
0,0
D.O.
ELUAT
INCORPORATION
FILTRAT
10 0
10 1
10 2
10 3
10 4
10 5
DILUTION

FIG.1

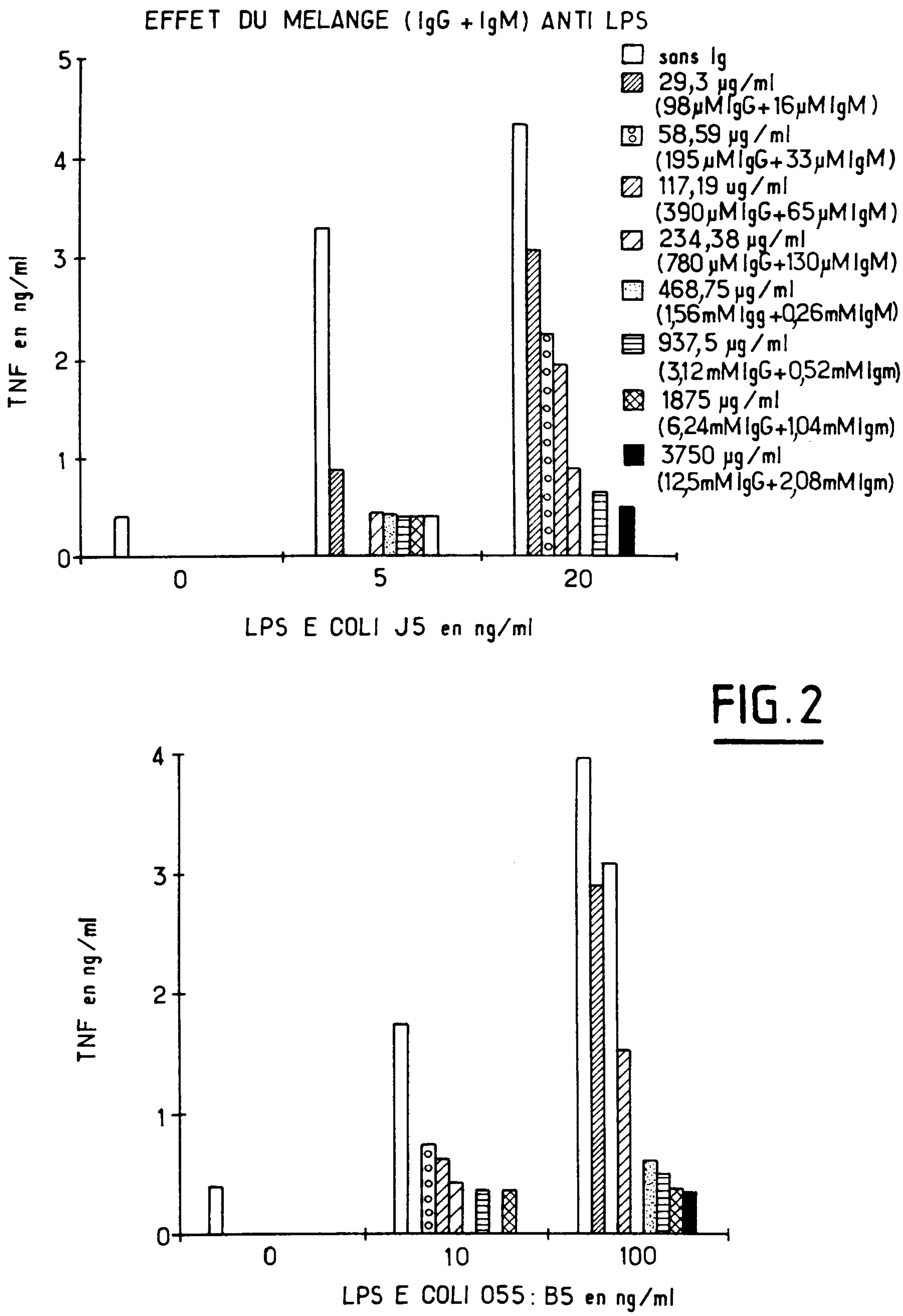
EFFET DU MELANGE (IgG + IgM) ANTI LPS
sans Ig
29,3 µg/ml (98µM IgG+16µM IgM)
58,59 µg/ml (195µM IgG+33µM IgM)
117,19 ug/ml (390µM IgG+65µM IgM)
234,38 µg/ml (780 µM IgG+130µM IgM)
468,75 µg/ml (1,56mM Igg +0,26mM IgM)
937,5 µg/ml (3,12mM IgG+0,52mM Igm)
1875 µg/ml (6,24mM IgG+1,04mM Igm)
3750 µg/ml (12,5mM IgG+2,08mM Igm)
TNF en ng/ml
LPS E COLI J5 en ng/ml
TNF en ng/ml
LPS E COLI 055: B5 en ng/ml

FIG.2

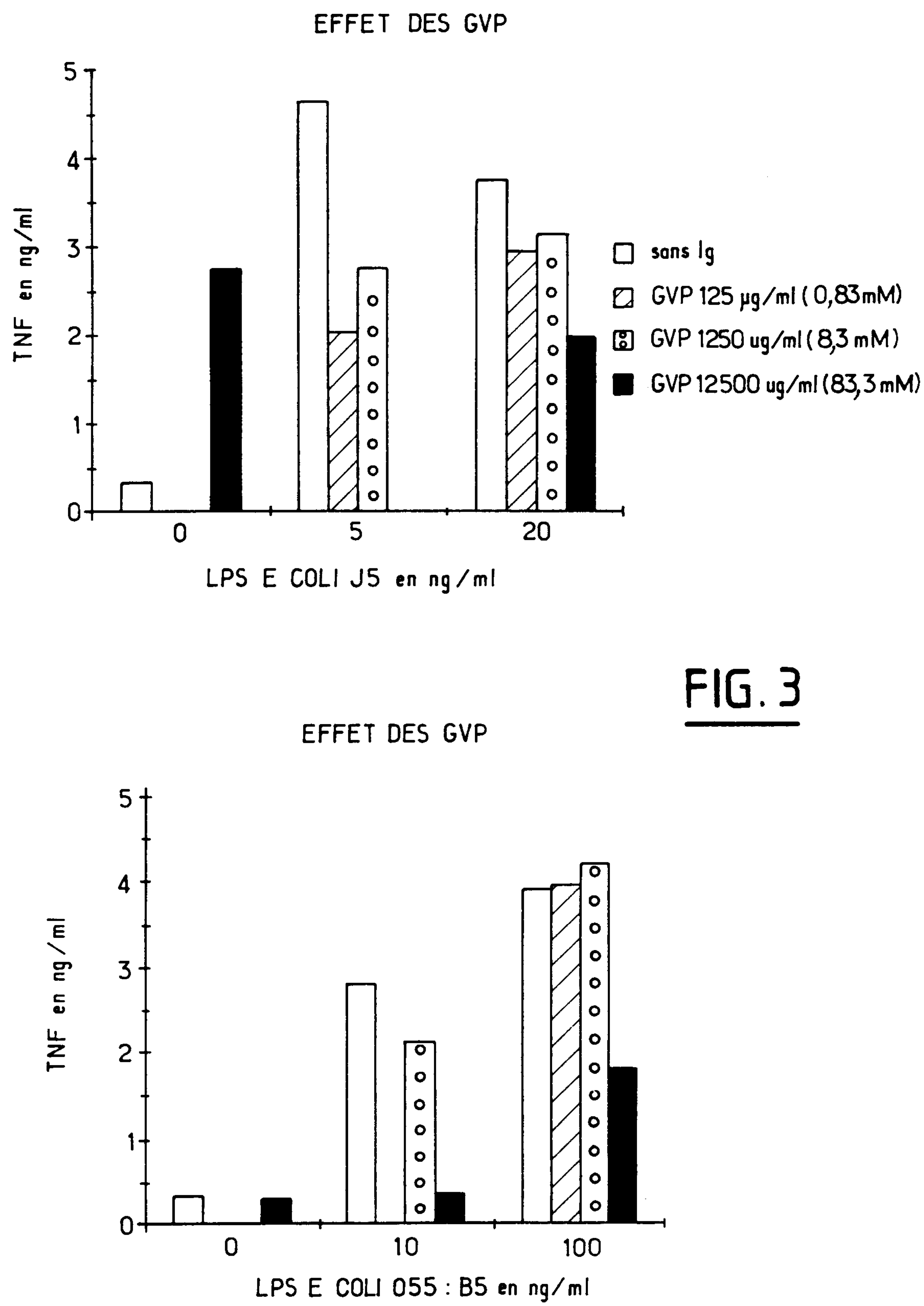
EFFET DES GVP
TNF en ng/ml
5
4
3
2
1
0
0
5
20
LPS E COLI J5 en ng/ml
sans Ig
GVP 125 µg/ml (0,83mM)
GVP 1250 ug/ml (8,3 mM)
GVP 12500 ug/ml (83,3 mM)
EFFET DES GVP
TNF en ng/ml
5
4
3
2
1
0
0
10
100
LPS E COLI 055 : B5 en ng/ml

FIG. 3

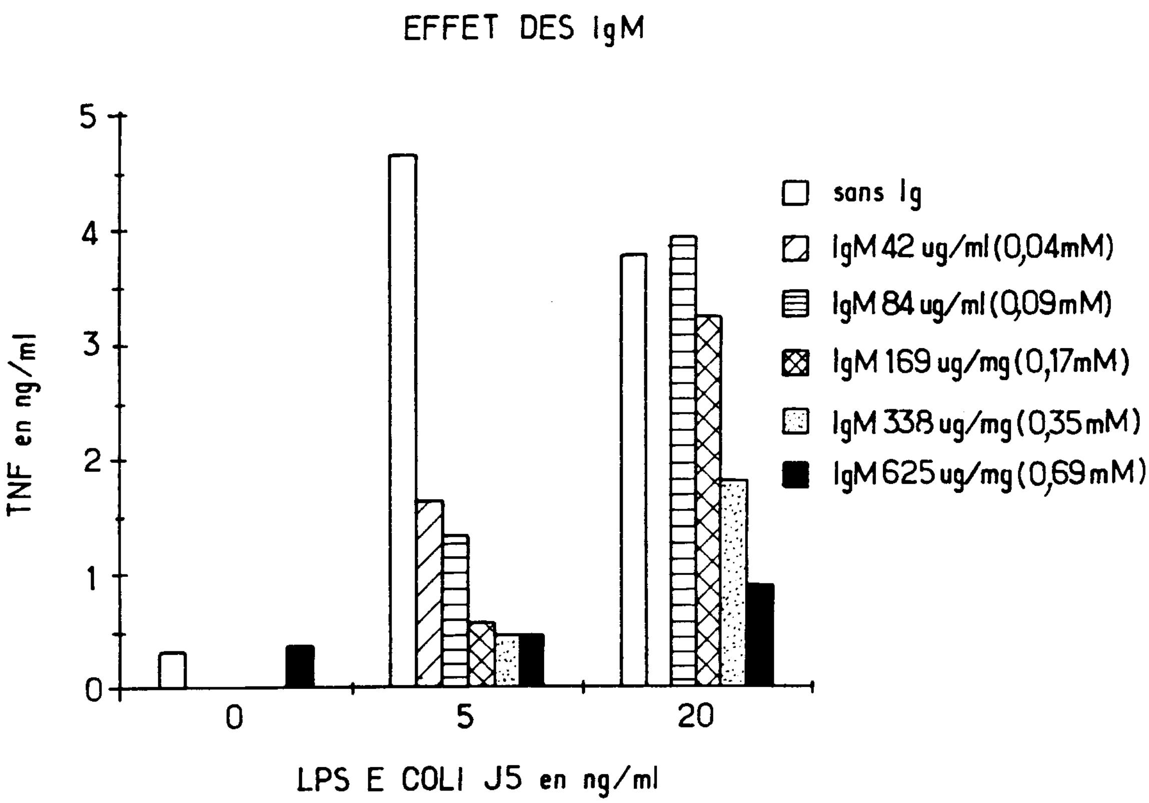
EFFET DES IgM
5
4
3
2
1
0
TNF en ng/ml
0
5
20
LPS E COLI J5 en ng/ml
sans Ig
IgM 42 ug/ml (0,04mM)
IgM 84 ug/ml (0,09mM)
IgM 169 ug/mg (0,17mM)
IgM 338 ug/mg (0,35mM)
IgM 625 ug/mg (0,69mM)

FIG. 4

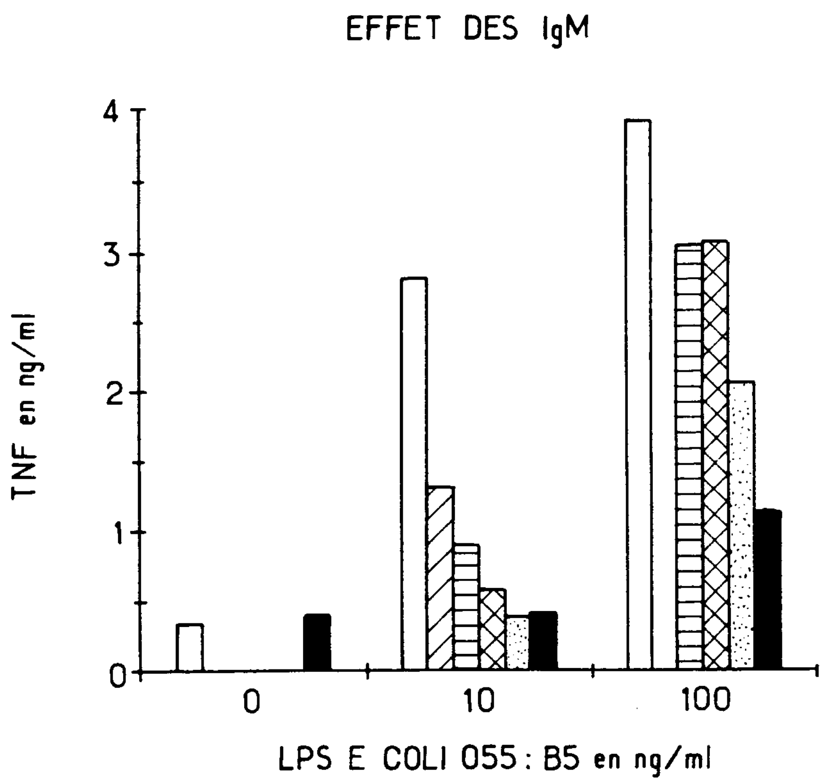
EFFET DES IgM
4
3
2
1
0
TNF en ng/ml
0
10
100
LPS E COLI 055 : B5 en ng/ml

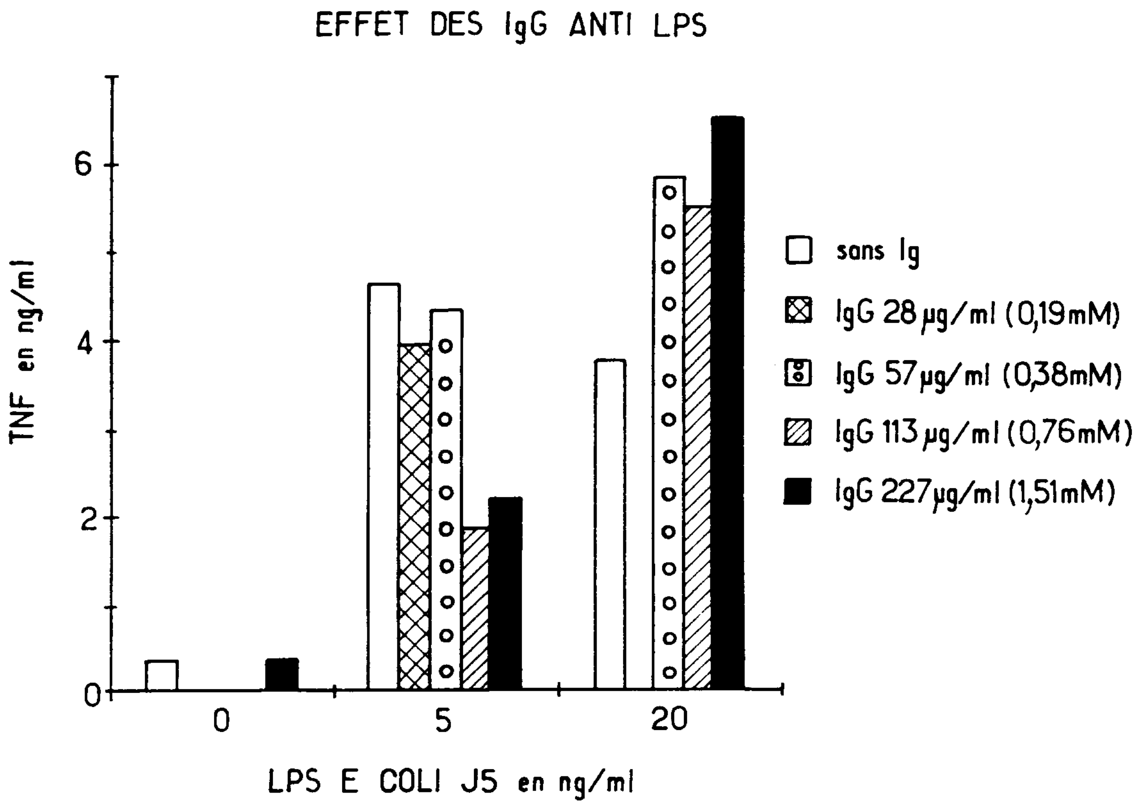
EFFET DES IgG ANTI LPS
TNF en ng/ml
0
2
4
6
0
5
20
LPS E COLI J5 en ng/ml
sans Ig
IgG 28 µg/ml (0,19mM)
IgG 57 µg/ml (0,38mM)
IgG 113 µg/ml (0,76mM)
IgG 227 µg/ml (1,51mM)

FIG.5

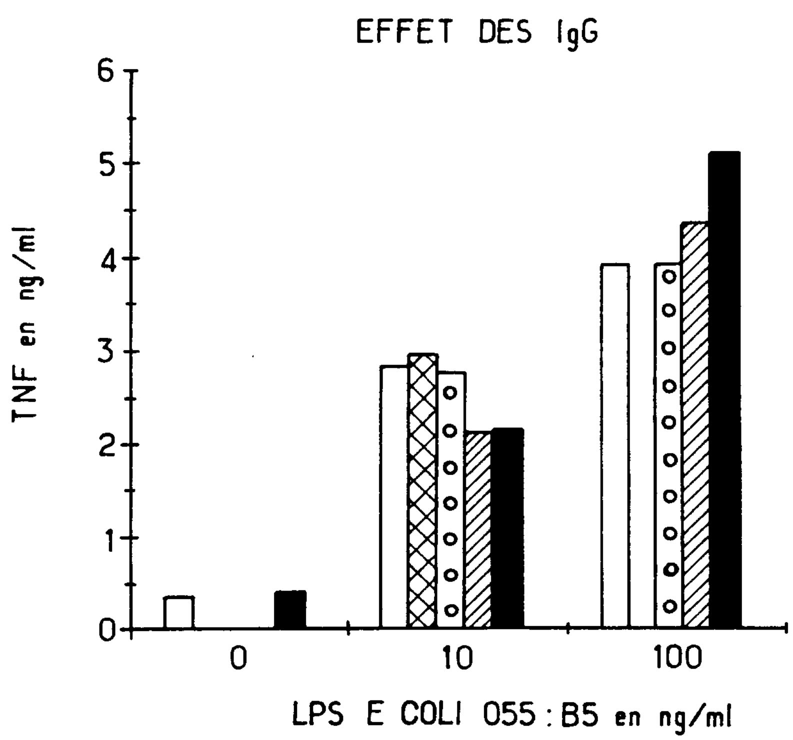
EFFET DES IgG
TNF en ng/ml
0
1
2
3
4
5
6
0
10
100
LPS E COLI 055 : B5 en ng/ml